(19) Europäisches Patentamt
European Patent Office
Office européen des brevets

(11) EP 4 538 305 A1

(12) EUROPEAN PATENT APPLICATION
published in accordance with Art. 153(4) EPC

(43) Date of publication:
16.04.2025 Bulletin 2025/16

(21) Application number: 23823702.8

(22) Date of filing: 31.05.2023

(51) International Patent Classification (IPC):
*C08F 20/30 (2006.01)* *C07C 69/54 (2006.01)*
*C09K 19/58 (2006.01)* *G02B 5/30 (2006.01)*
*G02C 7/10 (2006.01)* *G02C 7/12 (2006.01)*

(52) Cooperative Patent Classification (CPC):
**C07C 69/54; C08F 20/30; C09K 19/58; G02B 5/30; G02C 7/10; G02C 7/12**

(86) International application number:
**PCT/JP2023/020248**

(87) International publication number:
**WO 2023/243408 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022 JP 2022095190**

(71) Applicants:
- **Nippon Kayaku Kabushiki Kaisha
Tokyo 100-0005 (JP)**
- **Dejima Tech B.V.
6825 ML Arnhem (NL)**

(72) Inventors:
- **TANAKA Kouichi
Joetsu-shi, Niigata 944-0101 (JP)**
- **ERDHUISEN Erwin Wilhelmus Petrus
6825 (NL)**

(74) Representative: **Hentrich Patent- & Rechtsanwaltspartnerschaft mbB
Syrlinstraße 35
89073 Ulm (DE)**

# (54) ATROPISOMER OF 1,1'-BINAPHTHYL DERIVATIVE, POLYMERIZABLE LIQUID CRYSTAL COMPOUND, OPTICAL FILM, IMPAGE DISPLAY DEVICE, AND EYEWEAR

(57) The present invention relates to an atropisomer represented by the following general formula (1) and/or the general formula (2).

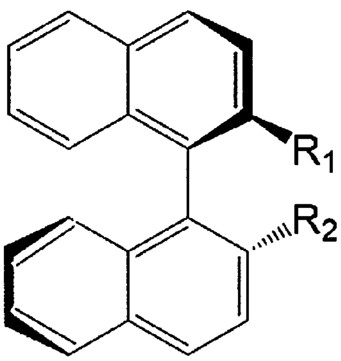

(1)

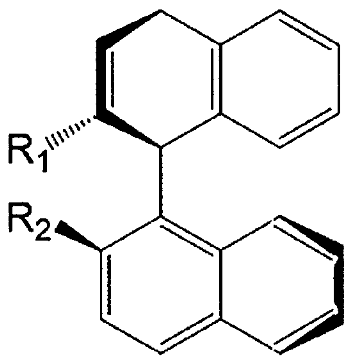

(2)

In the formula (1) and the formula (2), $R_1$ and $R_2$ each independently represent any one structure of (R-1) to (R-20), and at least one of $R_1$ and $R_2$ represents any one of (R-1) to (R-5), provided that $R_1$ and $R_2$ are not same except for a case where the structure is (R-1).

EP 4 538 305 A1

## Description

### Technical Field

The present invention relates to an atropisomer of a 1,1'-binaphthyl derivative, and a polymerizable liquid crystal composition, an optical film, an image display device, and eyewear using the same.

### Background Art

For an optical film as an optical compensation plate used for an image display device such as a liquid crystal display and eyewear such as polarizing sunglasses, cholesteric liquid crystals having a circularly polarizing separation function have been widely and conventionally used. Liquid crystal molecules in the cholesteric liquid crystal have helically twisted orientation, and have a property of selectively reflecting one of a left circularly polarized component or a right circularly polarized component, which correspond to a pitch of the helix. Such a cholesteric liquid crystal can be obtained by polymerizing a polymerizable liquid crystal composition containing a liquid-crystalline compound having a polymerizable functional group, such as a nematic liquid crystal, and a dopant agent similarly having a polymerizable functional group.

To allow the cholesteric liquid crystal to obtain the circularly polarizing separation function, a helical structure with a short pitch is required, and thus a liquid crystal composition containing a dopant agent with a strong helically twisting force in the liquid crystal is desirably used. The dopant agent can be used as a dopant that induces or reinforces the helical twist in the liquid crystal, and helical twisting power (HTP) is commonly used as an index to indicate helical orientation ability of the dopant agent.

The helical structure with a short pitch can be obtained by using a large amount of the dopant agent or by using a dopant agent having a high HTP value. The conventionally known dopant agents often exhibit a low HTP value, and thus a large amount of the dopant agent needs to be used to obtain the desired helical structure having a short pitch. However, there is concern that use of a large amount of the dopant agent adversely affects, for example, properties of the liquid crystal such as dielectric anisotropy, viscosity, and drive voltage. Therefore, development of a dopant agent that does not harmfully act on the properties of the liquid crystal has been demanded.

Patent Literature 1 discloses that a polymerizable chiral compound representing a predetermined structural formula has a high HTP value. However, the HTP value of the polymerizable chiral compound obtained by an actual test is up to about 65, and a polymerizable chiral compound having a higher HTP value is not described. Taking consideration of adverse effects on the properties of the liquid crystal, a concentration of the dopant agent relative to the liquid crystal compound is desired to be as low as possible.

### Document List

### Patent Literature

Patent Literature 1: Japanese Patent Application Publication No. 2013-87109

## Summary of Invention

### Technical Problem

An object of the present invention is to provide an atropisomer of a 1,1'-binaphthyl derivative having a high HTP value even with a small amount.

### Solution to Problem

An atropisomer according to an embodiment of the present invention is a compound represented by the following general formula (1) and/or general formula (2):

[Formula 1]

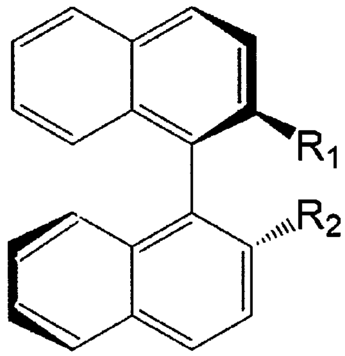

(1)

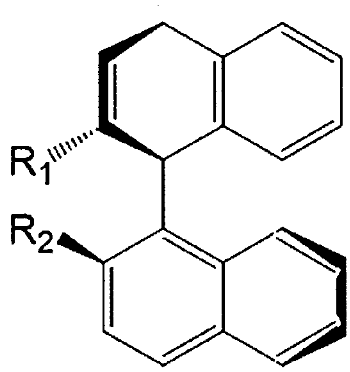

(2)

wherein $R_1$ and $R_2$ each independently represent any one structure of (R-1) to (R-20), and at least one of $R_1$ and $R_2$ represents any one of (R-1) to (R-5), provided that $R_1$ and $R_2$ are not same except for a case where the structure is (R-1),

[Formula 2]

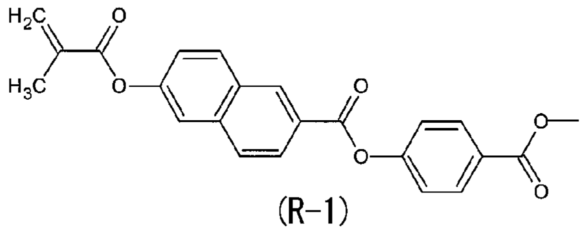

(R-1)

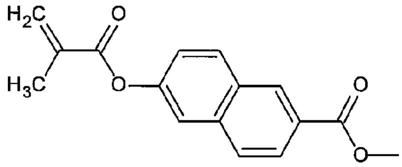

(R-2)

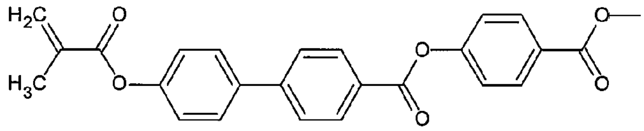

(R-3)

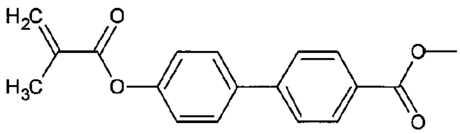

(R-4)

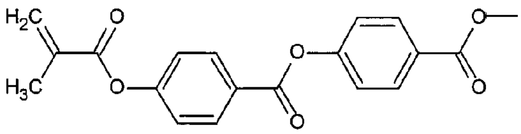

(R-5)

[Formula 3]

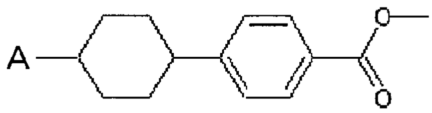

(R-6)

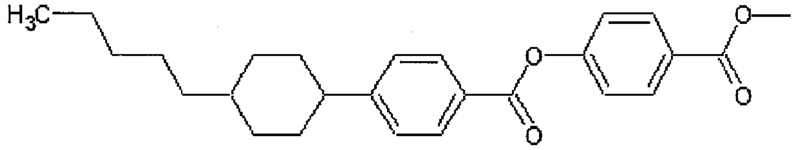

(R-7)

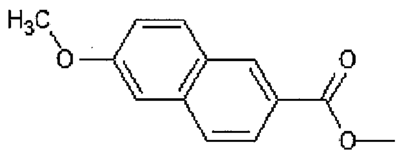

(R-8)

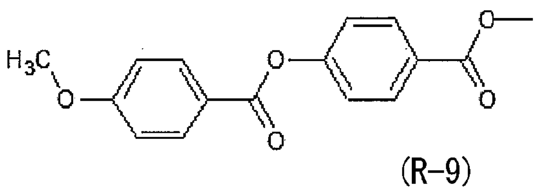

(R-9)

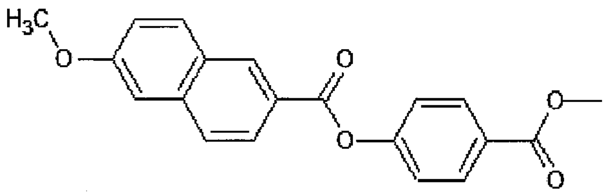

(R-10)

[Formula 4]

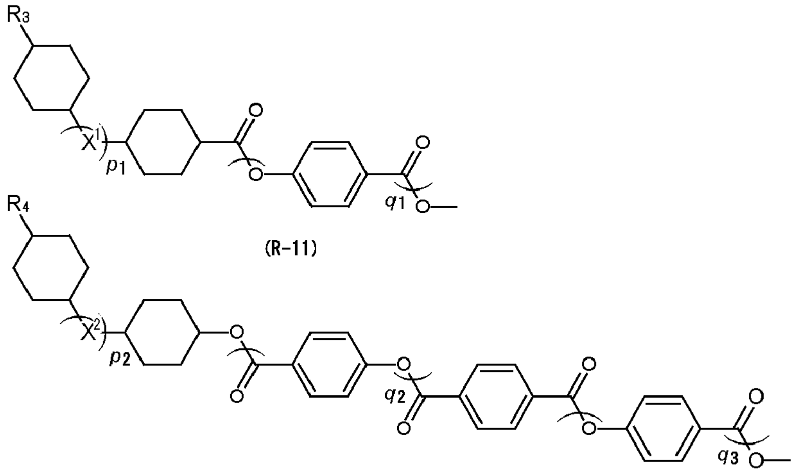

(R-11)

(R-12)

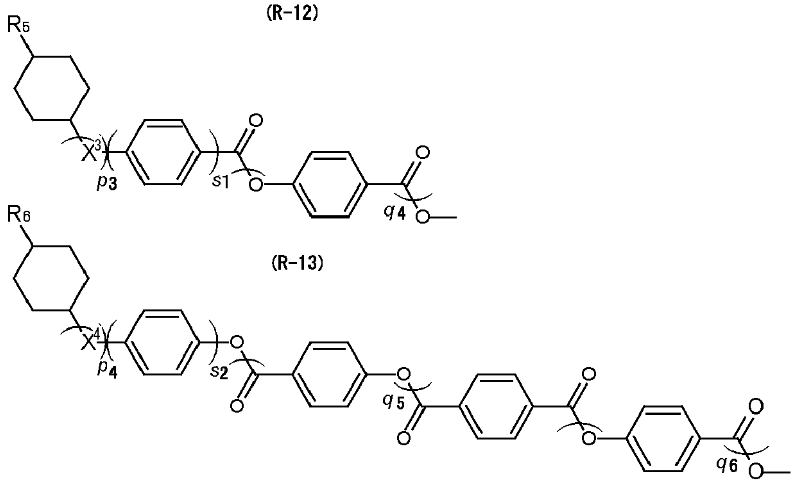

(R-13)

(R-14)

[Formula 5]

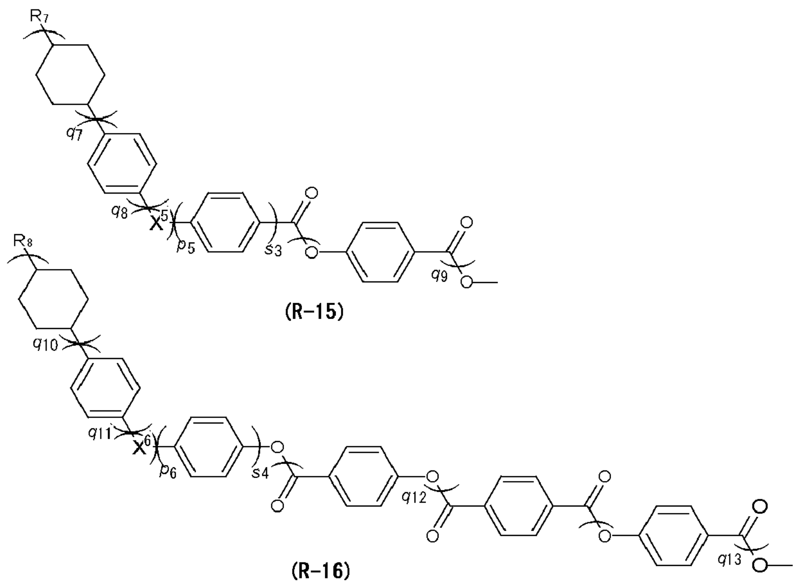

(R-15)

(R-16)

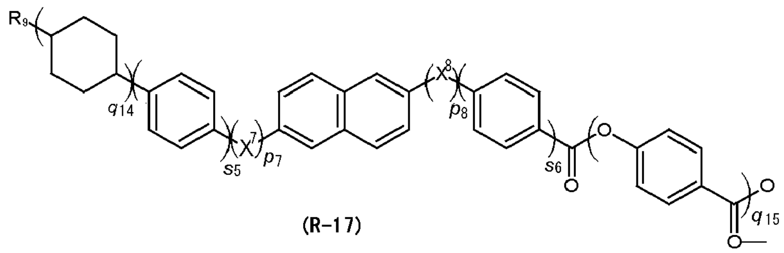

(R-17)

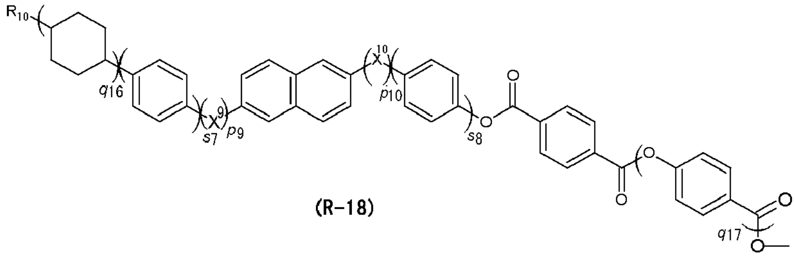

(R-18)

[Formula 6]

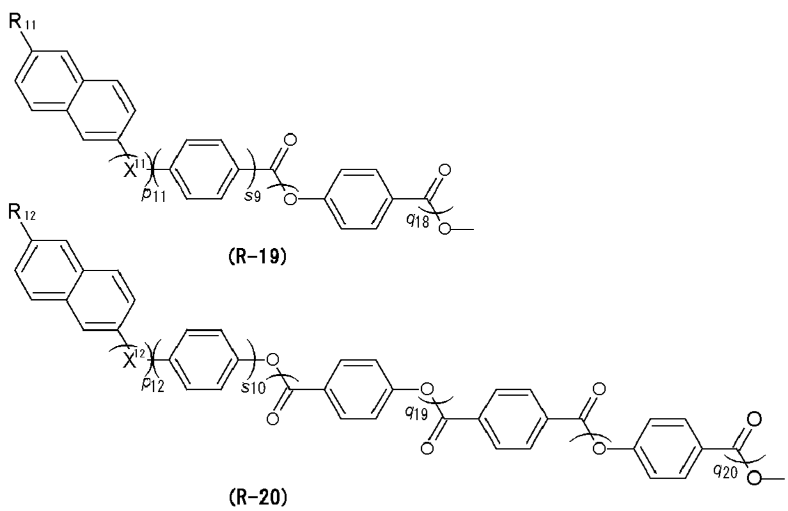

(R-19)

(R-20)

wherein A represents a $C_{1-12}$ alkyl group,

$R_3$ to $R_{12}$ each independently represent $-CnH_{2n+1}$, $-OCnH_{2n+1}$, CN, F, Cl, Br, $CF_3$, $Y-((CH_2)_{k1}-O)_m-$, $Y-(CH_2)_{k2}-O-C(O)-$, or $Y-(CH_2)_{k3}-COO-$,

Y represents $H_2C=C(Z_1)COO-$, $(H_2COCH)-CH_2-$, $H_2C=CH-CH_2-$, or $H_2C=CH-O-$,
$Z_1$ represents H or $CH_3$, k1 to k3 each independently represent an integer of 0 to 12,
m represents 0 or 1,
n represents an integer of 0 to 12,
$X^1$ to $X^{12}$ each independently represent -CH=CH-, -C≡C-, -HC=CH-COO-, -(O)CO-CH=CH-, $-CH_2-$, -O-, -C(O)-, $-CH_2CH_2-$, $-CH_2O-$, $-OCH_2-$, -COO-, - OC(O)-, $-C(CH_3)_2-$, -N=N-, -CH=N-, -N=CH-, -NHCO-, or -OCNH-,
p1 to p12 each independently represent 0 or 1,
q1 to q20 each independently represent 0, 1, or 2, and
s1 to s10 each independently represent 0, 1, 2, or 3.

In one embodiment of the present invention, $R_1$ and $R_2$ each independently represent any one structure of (R-1) to (R-10), and at least one of $R_1$ and $R_2$ represents any one of (R-1) to (R-4).
A polymerizable liquid crystal composition according to an embodiment of the present invention comprises: the aforementioned atropisomer; and a polymerizable liquid crystal compound.
An optical film according to an embodiment of the present invention comprises a cured film of the aforementioned polymerizable liquid crystal composition.
In one embodiment of the present invention, the aforementioned optical film has a selective reflection function.
An image display device according to an embodiment of the present invention comprises the aforementioned optical film.
Eyewear according to an embodiment of the present invention comprises the aforementioned optical film.

Effects of Invention

The present invention can achieve the atropisomer of the 1,1'-binaphthyl derivative having a high HTP value even with a small amount.

Description of Embodiments

Hereinafter, the atropisomer according to the present embodiment, and the polymerizable liquid crystal composition, the optical film, the image display device, and the eyewear using the same will be described in detail.

<Atropisomer>

The atropisomer according to the present embodiment is a compound represented by the following general formula (1) and/or general formula (2). These compounds are atropisomers of a 1,1'-binaphthyl derivative. The compound represented by the general formula (1) is an R-isomer, and the compound represented by the general formula (2) is an S-isomer. The atropisomer may be any one of the compounds represented by the general formula (1) and the general formula (2), or may contain both of them.

[Formula 7]

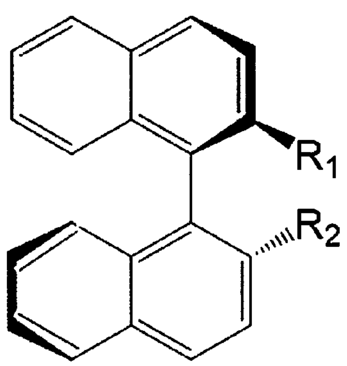
(1)

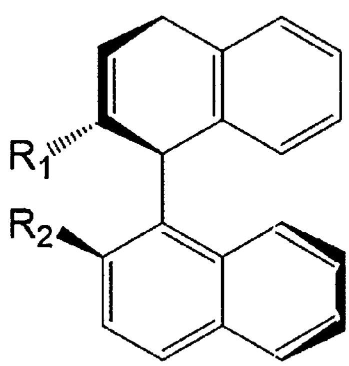
(2)

In the compounds represented by the general formula (1) and the general formula (2), $R_1$ and $R_2$ each independently represent any one structure of the following (R-1) to (R-20), and at least one of $R_1$ and $R_2$ represents

any one of (R-1) to (R-5). Provided that $R_1$ and $R_2$ are not same except for a case where the structure is (R-1), that is, $R_1$ and $R_2$ have structures different from each other except for a case where both $R_1$ and $R_2$ are each (R-1). Since at least one of $R_1$ and $R_2$ has a polymerizable group represented by $H_2C=C(CH_3)$-, the compounds represented by the general formula (1) and the general formula (2) exhibit a polymerizable property, and function as a dopant agent for a polymerizable liquid crystal compound, which is a similar liquid crystal component.

[Formula 8]

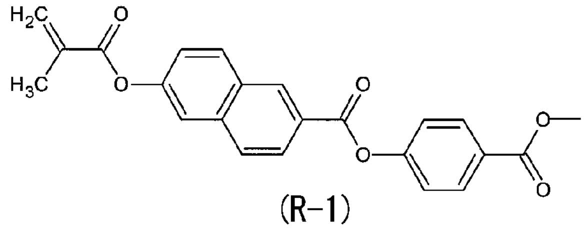

(R-1)

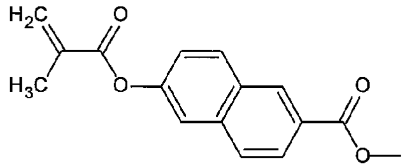

(R-2)

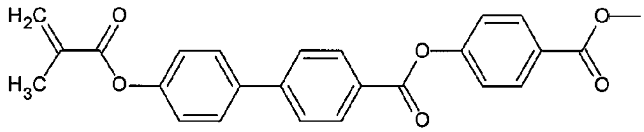

(R-3)

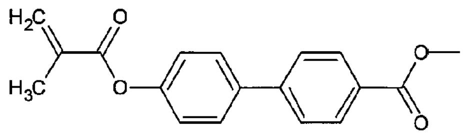

(R-4)

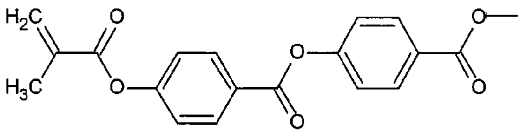

(R-5)

[Formula 9]

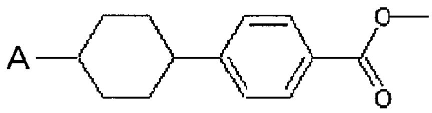

(R-6)

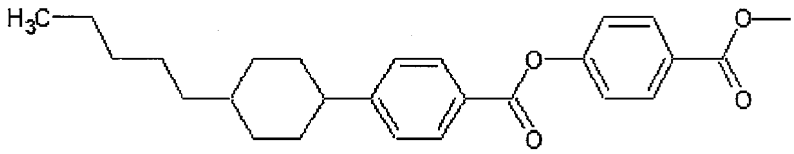

(R-7)

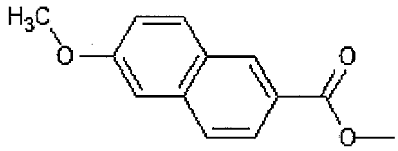

(R-8)

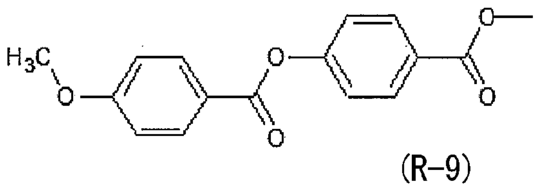

(R-9)

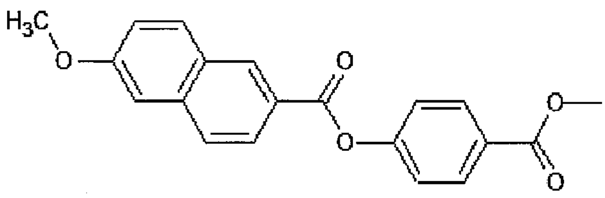

(R-10)

[Formula 10]

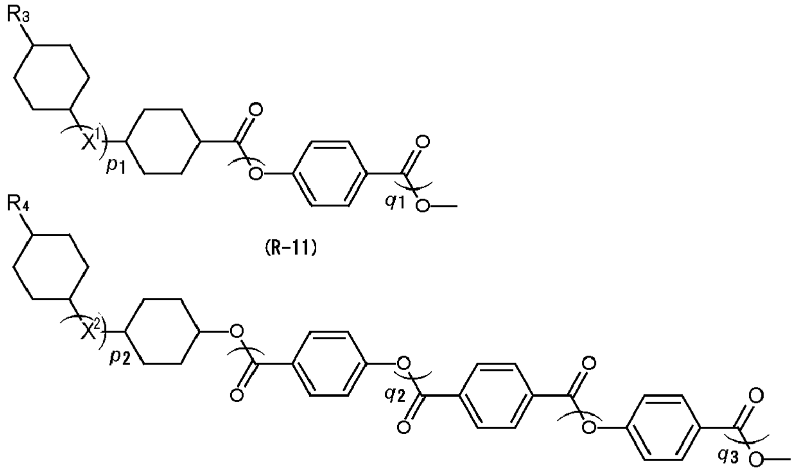

(R-11)

(R-12)

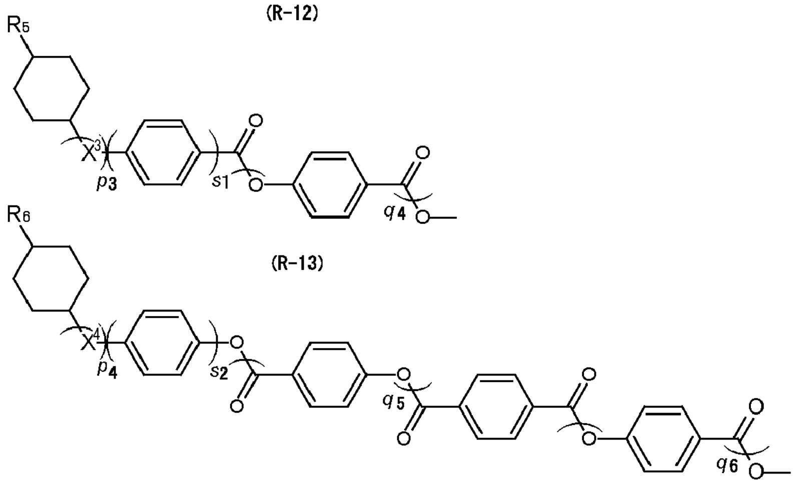

(R-13)

(R-14)

[Formula 11]

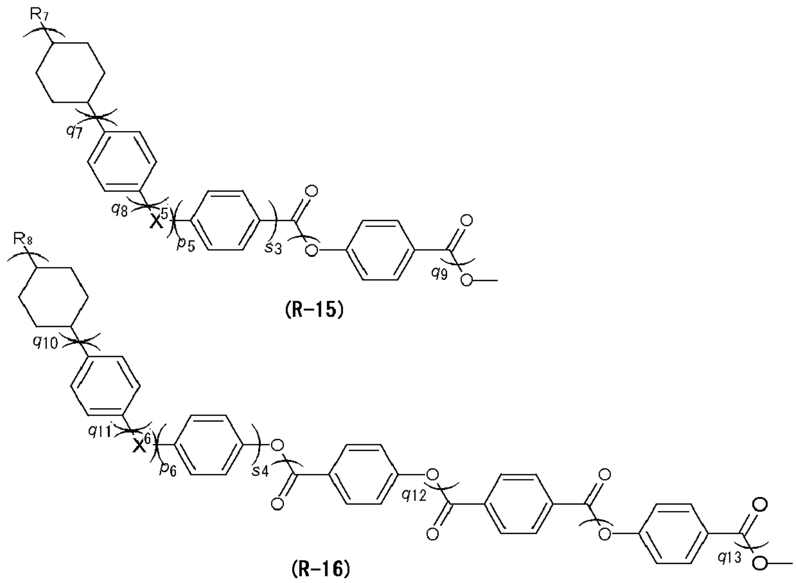

(R-15)

(R-16)

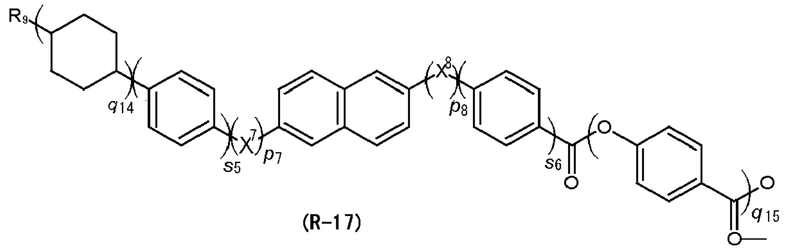

(R-17)

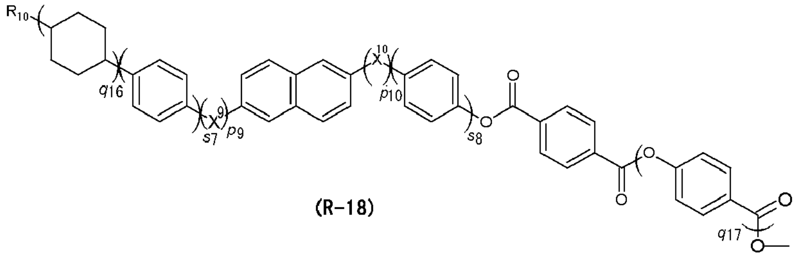

(R-18)

[Formula 12]

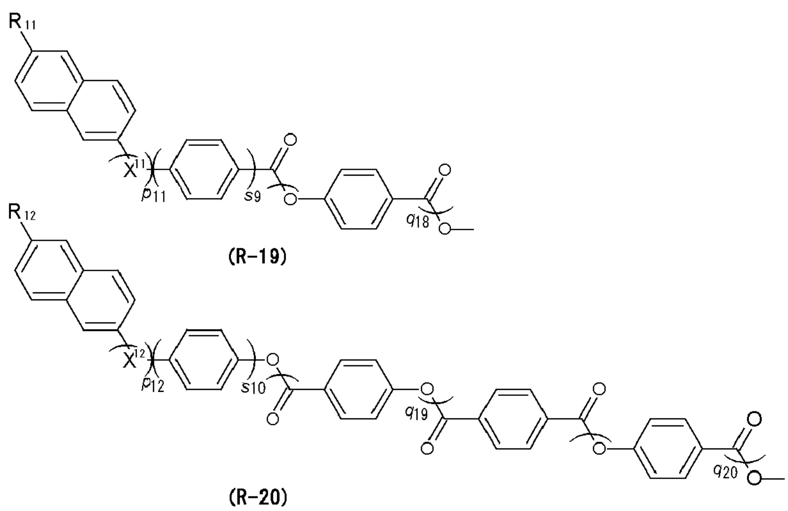

(R-19)

(R-20)

In the structure of the (R-6), A represents a $C_{1-12}$ alkyl group, and preferably represents a $C_{3-6}$ alkyl group. The $C_{1-12}$ alkyl group may be linear or branched. Examples of such a $C_{1-12}$ alkyl group include methyl group, ethyl group, n-propyl group, i-propyl group, n-butyl group, i-butyl group, sec-butyl group, tert-butyl group, pentyl group, hexyl group,

heptyl group, octyl group, nonyl group, decyl group, undecyl group, and dodecyl group, and is preferably n-propyl group or pentyl group.

In the structures of the (R-11) to (R-20), $R_3$ to $R_{12}$ each independently represent $-CnH_{2n+1}$, $-OCnH_{2n+1}$, CN, F, Cl, Br, $CF_3$, $Y-((CH_2)_{k1}-O)_m-$, $Y-(CH_2)_{k2}-OC(O)-$, or $Y-(CH_2)_{k3}-COO-$,

Y represents $H_2C=C(Z_1)COO-$, $(H_2COCH)-CH_2-$, $H_2C=CH-CH_2-$, or $H_2C=CH-O-$, and preferably $H_2C=C(Z_1)COO-$,
$Z_1$ represents H or $CH_3$, preferably $CH_3$,
k1 to k3 each independently represent an integer of 0 to 12, preferably 0, m represents 0 or 1, preferably 0,
n represents an integer of 0 to 12, preferably 1, 3, 4, 5, or 6, and in the structures of (R-19) and (R-20), n=1 is particularly preferable,
$X^1$ to $X^{12}$ each independently represent -CH=CH-, -C≡C-, -HC=CH-COO-, -(O)CO-CH=CH-, $-CH_2-$, -O-, -C(O)-, $-CH_2CH_2-$, $-CH_2O-$, $-OCH_2-$, -COO-, - OC(O)-, $-C(CH_3)_2-$, -N=N-, -CH=N-, -N=CH-, -NHCO-, or -OCNH-, preferably -COO-,
p1 to p12 each independently represent 0 or 1, preferably 0,
q1 to q20 each independently represent 0, 1, or 2, preferably 1, and provided that, in the structures of (R-15) and (R-16), q7 and q10 particularly preferably represent each 0, and
s1 to s10 each independently represent 0, 1, 2, or 3, preferably 1, and provided that, in the structures of (R-19) and (R-20), s9 and s10 particularly preferably represent each 0.

It is preferable that $R_1$ and $R_2$ each independently represent any one structure of (R-1) to (R-10), and at least one of $R_1$ and $R_2$ represents any one of (R-1) to (R-4). When one of $R_1$ and $R_2$ represents the structure of (R-11) to (R-20), preferably represents (R-11), (R-13), (R-15), (R-17), or (R-19).

In the atropisomer according to the present embodiment, $R_1$ and $R_2$ are unsymmetrical each other except for a case where both $R_1$ and $R_2$ represent each (R-1) in the compounds represented by the general formula (1) and the general formula (2), and $R_1$ and $R_2$ are not integrated with each other to form one backbone structure. Since the compounds represented by the general formula (1) and the general formula (2) have such a specific structure, the atropisomer exhibiting a high HTP value even with a small amount can be obtained. An optical film obtained by using a polymerizable liquid crystal composition containing the atropisomer having such a property as a dopant agent can exhibit a center reflection wavelength with a broad band according to a content of the atropisomer, and in particular, can broaden the reflection band in a visible light region.

<Method for manufacturing atropisomer>

The atropisomer according to the present embodiment can be manufactured by a synthesis method described below, for example.

<Manufacture Example 1: Manufacture of compound represented by general formula (2) in which $R_1$ and $R_2$ are symmetrical>

(S)-1,1'-Bi-2-naphthol (S-Binol), dimethylaminopyridine (DMAP), and 4-({6-[(2-methylprop-2-enoyl)oxy]naphthalene-2-carbonyl}oxy)benzoic acid (Ma-NP-BA) are added into a reaction vessel, and stirred in dichloromethane (DCM). Into the obtained suspension liquid, 1-(3-dimethylaminopropyl)-3-ethylcarbodiimide hydrochloride (EDC) is added little by little with a constant interval to obtain a compound in which S-Binol is di-esterified with Ma-NP-BA, that is, a compound represented by the following general formula (2.1) in which $R_1$ and $R_2$ each represent the structure (R-1).

[Formula 13]

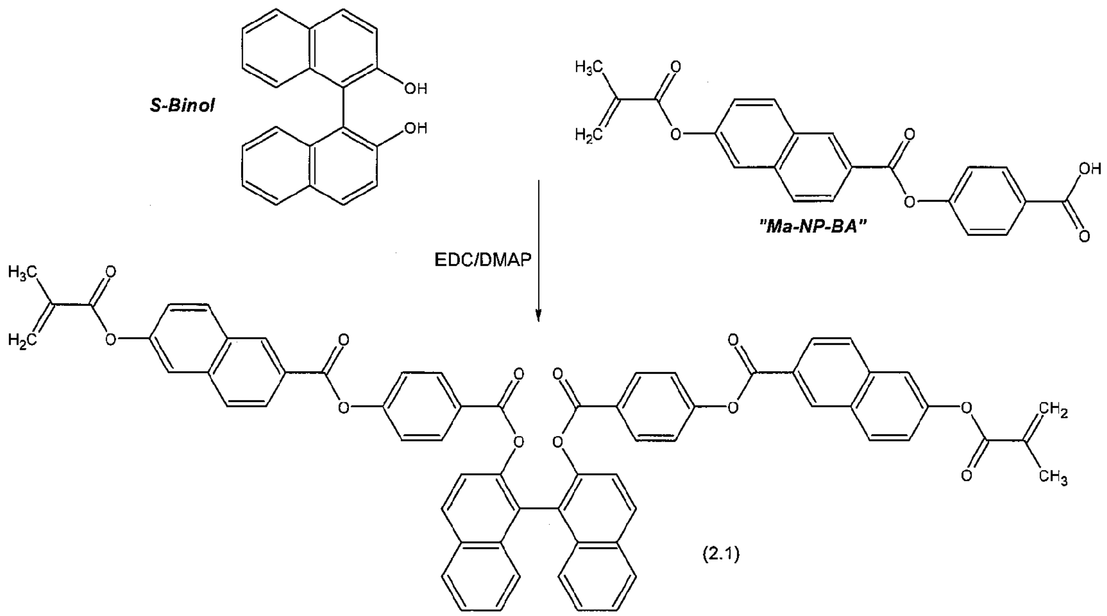


(2.1)

<Manufacture Example 2: Manufacture of compound represented by general formula (2) in which $R_1$ and $R_2$ are unsymmetrical>

(Step 1)

S-Binol, DMAP, and 4-(trans-4-pentylcyclohexyl)benzoic acid (5PCA) are added into a reaction vessel, and stirred in DCM. Into the obtained suspension liquid, EDC is added little by little with a constant interval to synthesize a compound in which S-Binol is mono-esterified with 5PCA (S-Binol monoester of 5PCA).

(Step 2)

Into a solution containing the S-Binol monoester of 5PCA, 4'-[(2-methylprop-2-enoyl)oxy][1,1'-biphenyl]-4-carboxylic acid (Ma-BPh-A) and EDC are added to obtain a compound in which S-Binol is di-esterified with 5PCA and Ma-BPh-A, that is, a compound represented by the general formula (2.2) in which one of $R_1$ and $R_2$ represents the structure (R-6) and the other represents the structure (R-4).

[Formula 14]

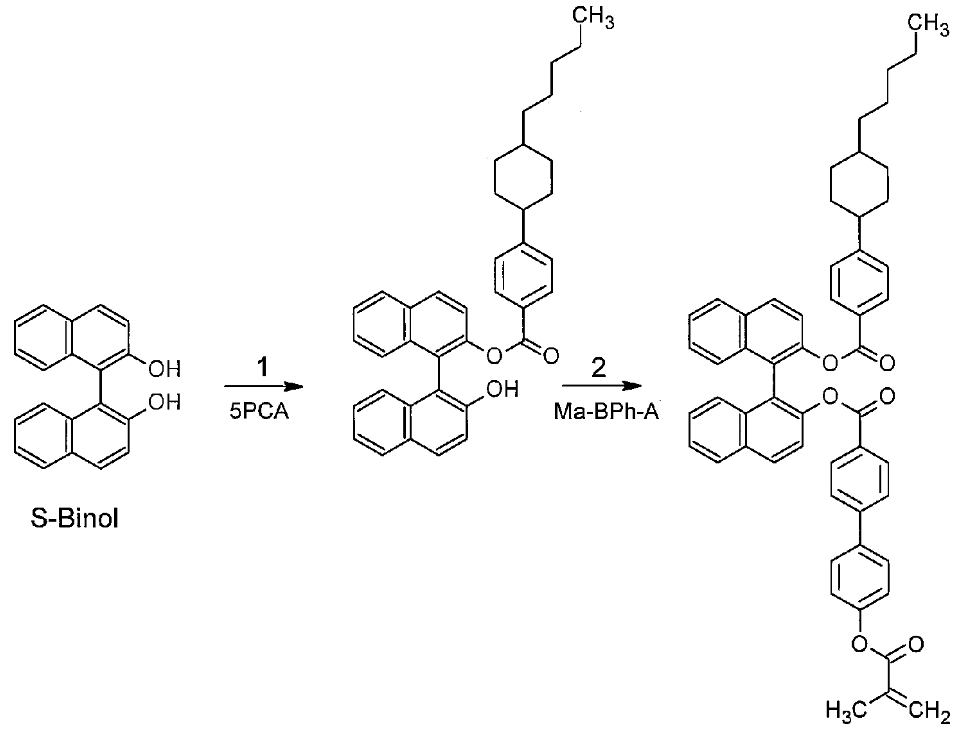


S-Binol

(2. 2)

<Polymerizable liquid crystal composition>

The polymerizable liquid crystal composition according to the present embodiment contains a polymerizable liquid crystal compound and the aforementioned atropisomer as a dopant agent. In the polymerizable liquid crystal composition, a polymerization initiator to polymerize these to enhance curing of the polymerizable liquid crystal composition, a solvent to dissolve these, and optional additives are contained. Such a polymerizable liquid crystal composition can be produced by dissolving the liquid crystal components of the polymerizable liquid crystal compound and the aforementioned atropisomer in the solvent, and adding the polymerization initiator and the optional additives into the obtained solution. When the aforementioned atropisomer is added into the polymerizable liquid crystal compound, the polymerizable liquid crystal compound forms a cholesteric liquid crystal state, and the liquid crystal molecules are helically oriented. By helically orientating the liquid crystal molecules, a selective reflection function in which a circularly polarized component with a wavelength of light corresponding to the helical pitch is selectively reflected is imparted in an optical element produced by using the polymerizable liquid crystal composition.

The polymerizable liquid crystal compound is a compound that exhibits liquid crystallinity within a predetermined temperature range and concentration range, and that is polymerized by ultraviolet ray or heat while keeping the orientation state to be able to fix the orientation state. Such a polymerizable liquid crystal compound is preferably a polymerizable liquid crystal monomer having a polymerizable group in the molecule. Examples of the polymerizable group include (meth) acryloyl group, vinyl group, chalcone group, cinnamoyl group, and epoxy group. To exhibit the liquid crystallinity, mesogenic group is preferably present in the molecule, and the mesogenic group means a substituent having, for example, a rod shape such as biphenyl group, terphenyl group, (poly)phenyl benzoate group, (poly)ether group, benzylideneaniline group, or acenaphthoquinoxaline group, a plate shape, or a disk shape such as triphenylene group, phthalocyanine group, or aza-crown group, that is, a group having ability to induce behavior of the liquid crystal phase. The liquid crystal compounds having the rod-shaped or the plate-shaped substituent are already known as a calamitic liquid crystal in this technical field. Specific examples of such a polymerizable liquid crystal monomer include: polymerizable liquid crystal compounds described in Japanese Patent Application Publication No. 2003-315556, Japanese Patent Application Publication No. 2004-29824, and Japanese Patent No. 5463666; and polymerizable nematic liquid crystal monomers such as PALIOCOLOR series (manufactured by BASF SE) and RMM series (manufactured by Merck KGaA). The polymerizable liquid crystal compound may be used singly, or may be used in combination of two or more thereof.

Since the aforementioned atropisomer that functions as the dopant agent can twistedly orient the aforementioned polymerizable liquid crystal compound clockwise or counterclockwise, the polymerizable liquid crystal composition exhibiting the cholesteric liquid crystal phase can be obtained. By the polymerization reaction between the aforementioned polymerizable liquid crystal compound and the aforementioned atropisomer, a polymer having a repeating unit induced

from the polymerizable liquid crystal compound and a repeating unit induced from the compound represented by the general formula (1) and/or the general formula (2) is formed. A content of the atropisomer contained in the polymerizable liquid crystal composition is preferably 0.05 parts by mass or more and 15 parts by mass or less relative to 100 parts by mass of the polymerizable liquid crystal compound used in combination, and a lower limit of the content is more preferably 0.1 part by mass, and further preferably 1.0 part by mass. An upper limit of the content of the atropisomer contained in the polymerizable liquid crystal composition is more preferably 10 parts by mass or less, and further preferably 5.0 parts by mass or less relative to 100 parts by mass of the polymerizable liquid crystal compound. Therefore, the content of the atropisomer contained in the polymerizable liquid crystal composition is more preferably 0.1 part by mass or more and 10 parts by mass or less, and further preferably 1.0 part by mass or more and 5.0 parts by mass or less relative to 100 parts by mass of the polymerizable liquid crystal compound. As the content of the polymerizable atropisomer in the liquid crystal composition is lower, the effect on the liquid crystallinity can be reduced.

The polymerizable liquid crystal composition may contain a polymerizable compound that can react with the polymerizable liquid crystal compound and that has no liquid crystallinity. Examples of such a polymerizable compound include ultraviolet-ray curable resins. Examples of the ultraviolet-ray curable resins include dipentaerythritol hexa(meth)acrylate, a reaction product of dipentaerythritol penta(meth)acrylate and 1,6-hexamethylene diisocyanate, a reaction product of triisocyanate having isocyanurate ring and pentaerythritol tri(meth)acrylate, a reaction product of pentaerythritol tri(meth)acrylate and isophorone diisocyanate, dipentaerythritol penta(meta)acrylate, dipentaerythritol tetra(meth)acrylate, pentaerythritol tetra(meth)acrylate, pentaerythritol tri(meth)acrylate, trimethylolpropane tri(meth)acrylate, ditrimethylolpropane tetra(meth)acrylate, tris(acryloxyethyl) isocyanurate, tris(methacryloxyethyl) isocyanurate, a reaction product of glycerol triglycidyl ether and (meth)acrylic acid, caprolactone-modified tris(acryloxyethyl) isocyanurate, a reaction product of trimethylolpropane triglycidyl ether and (meth)acrylic acid, triglycerol di(meth)acrylate, a reaction product of propylene glycol diglycidyl ether and (meth)acrylic acid, polypropylene glycol di(meth)acrylate, tripropylene glycol di(meth)acrylate, polyethylene glycol di(meth)acrylate, tetraethylene glycol di(meth)acrylate, triethylene glycol di(meth)acrylate, pentaerythritol di(meth)acrylate, a reaction product of 1,6-hexanediol diglycidyl ether and (meth)acrylic acid, 1,6-hexanediol di(meth)acrylate, glycerol di(meth)acrylate, a reaction product of ethylene glycol diglycidyl ether and (meth)acrylic acid, a reaction product of diethylene glycol diglycidyl ether and (meth)acrylic acid, bis(acryloxyethyl) hydroxyethyl isocyanurate, bis(methacryloxyethyl) hydroxyethyl isocyanurate, a reaction product of bisphenol A diglycidyl ether and (meth)acrylic acid, tetrahydrofurfuryl (meth)acrylate, caprolactone-modified tetrahydrofurfuryl (meth)acrylate, 2-hydroxyethyl (meth)acrylate, 2-hydroxypropyl (meth)acrylate, polypropylene glycol (meth)acrylate, polyethylene glycol (meth)acrylate, phenoxyhydroxypropyl (meth)acrylate, acryloylmorpholine, methoxypolyethylene glycol (meth)acrylate, methoxytetraethylene glycol (meth)acrylate, methoxytriethylene glycol (meth)acrylate, methoxyethylene glycol (meth)acrylate, methoxyethyl (meth)acrylate, glycidyl (meth)acrylate, glycerol (meth)acrylate, ethylcarbitol (meth)acrylate, 2-ethoxyethyl (meth)acrylate, N,N-dimethylaminoethyl (meth)acrylate, 2-cyanoethyl (meth)acrylate, a reaction product of butyl glycidyl ether and (meth)acrylic acid, butoxytriethylene glycol (meth)acrylate, and butanediol mono(meth)acrylate. These may be used singly, or a plurality of these may be mixed for use. These ultraviolet-ray curable resins having no liquid crystallinity are added in the liquid crystal composition at such an extent that the polymerizable liquid crystal composition does not lose the liquid crystallinity, and the addition amount is preferably 0.1 part by mass or more and 25 parts by mass or less, and more preferably 0.5 parts by mass or more and 10 parts by mass or less relative to 100 parts by mass of the polymerizable liquid crystal compound.

To cure the polymerizable liquid crystal composition with the polymerization reaction, the polymerizable liquid crystal composition may contain a polymerization initiator. Since the polymerizable liquid crystal composition typically proceeds the curing reaction with ultraviolet ray irradiation, the polymerization initiator to be used is preferably a photopolymerization initiator that can initiate the polymerization reaction with the ultraviolet ray irradiation. The photopolymerization initiator is not particularly limited, and examples thereof include: acetophenone compounds such as 2-methyl-1-[4-(methylthio)phenyl]-2-morpholinopropan-1-one (manufactured by IGM Resins B.V., "Omnirad 907"), 1-hydroxycyclohexyl phenyl ketone (manufactured by IGM Resins B.V., "Omnirad 184"), 4-(2-hydroxyethoxy)phenyl (2-hydroxy-2-propyl) ketone (manufactured by IGM Resins B.V., "Omnirad 2959"), 1-(4-dodecylphenyl)-2-hydroxy-2-methylpropan-1-one (manufactured by Merck KGaA, "Darocur 953"), 1-(4-isopropylphenyl)-2-hydroxy-2-methylpropan-1-one (manufactured by Merck KGaA, "Darocur 1116"), 2-hydroxy-2-methyl-1-phenylpropan-1-one (manufactured by IGM Resins B.V., "Omnirad 1173"), and diethoxyacetophenone;

benzoin compounds such as benzoin, benzoin methyl ether, benzoin ethyl ether, benzoin isopropyl ether, benzoin isobutyl ether, and 2,2-dimethoxy-2-phenylacetophenone (manufactured by IGM Resins B.V., "Omnirad 651");
benzophenone compounds such as benzoylbenzoic acid, methyl benzoylbenzoate, 4-phenylbenzophenone, hydroxybenzophenone, 4-benzoyl-4'-methyldiphenyl sulfide, and 3,3'-dimethyl-4-methoxybenzophenone (manufactured by Nippon Kayaku Co., Ltd., "KAYACURE MBP");
phosphine oxide compounds such as 2,4,6-trimethylbenzoyldiphenylphosphine oxide (manufactured by IGM Resins B.V., "Omnirad TPO") and bis(2,4,6-trimethylbenzoyl)phenylphosphine oxide (manufactured by IGM Resins B.V.,

"Omnirad 819"); and
thioxanthone compounds such as thioxanthone, 2-chlorothioxanthone (manufactured by Nippon Kayaku Co., Ltd., "KAYACURE CTX"), 2-methylthioxanthone, 2,4-dimethylthioxanthone (manufactured by Nippon Kayaku Co., Ltd., "KAYACURE RTX"), isopropylthioxanthone, 2,4-dichlorothioxanthone (manufactured by Nippon Kayaku Co., Ltd., "KAYACURE CTX"), 2,4-diethylthioxanthone (manufactured by Nippon Kayaku Co., Ltd., "KAYACURE DETX"), and 2,4-diisopropylthioxanthone (manufactured by Nippon Kayaku Co., Ltd., "KAYACURE DITX"). These photopolymerization initiators may be used singly, or may be used in combination of two or more thereof.

When the benzophenone compound or the thioxanthone compound is used as the photopolymerization initiator, a reaction auxiliary is preferably used in combination to enhance the photopolymerization reaction. The reaction auxiliary is not particularly limited, and examples thereof include amine compounds such as triethanolamine, methyldiethanolamine, triisopropanolamine, n-butylamine, N-methyldiethanolamine, diethylaminoethyl methacrylate, Michler's Ketone, 4,4'-diethylaminophenone, ethyl 4-dimethylaminobenzoate, 2-butoxyethyl 4-(dimethylamino)benzoate, and isoamyl 4-(dimethylamino)benzoate.

Addition amounts of the photopolymerization initiator and the reaction auxiliary are preferably within a range not affecting the liquid crystallinity of the polymerizable liquid crystal composition, preferably 0.05 parts by mass or more and 10 parts by mass or less, and more preferably 0.1 part by mass or more and 5 parts by mass or less relative to 100 parts by mass of the compounds curable with ultraviolet ray in the polymerizable liquid crystal composition. The addition amount of the auxiliary is preferably double or more of the photopolymerization initiator on a mass basis.

The polymerizable liquid crystal composition further contains a solvent. Such a solvent is not particularly limited as long as it can dissolve the polymerizable liquid crystal compound, the dopant agent, etc. to be used, and examples thereof include methyl ethyl ketone, toluene, methyl isobutyl ketone, cyclopentanone, acetone, xylene, and anisole. These solvents may be added at any proportion, and only one type thereof may be added, or a plurality of the solvents may be used in combination. These solvents are dried and removed in a drying zone such as an oven and a film coater line.

The polymerizable liquid crystal composition may further contain, as necessary, additives such as a leveling agent, an ultraviolet ray absorbent, a light stabilizer, an antioxidant, a polymerization inhibitor, a crosslinking agent, a plasticizer, and a flowing regulator at any proportion. Examples of the leveling agent include a fluorine-based compound, a silicone-based compound, and an acrylic compound. Examples of the ultraviolet ray absorbent include a benzotriazole-based compound, a benzophenone-based compound, and a triazine-based compound, examples of the light stabilizer include a hindered amine-based compound and a benzoate-based compound, and examples of the antioxidant include a phenol-based compound. Examples of the polymerization inhibitor include methoquinone, methylhydroquinone, and hydroquinone, and examples of the crosslinking agent include a polyisocyanate and a melamine compound. Examples of the plasticizer include: phthalate esters such as dimethyl phthalate and diethyl phthalate; trimellitate esters such as tris(2-ethylhexyl) trimellitate; aliphatic dibasic acid esters such as dimethyl adipate and dibutyl adipate; orthophosphate esters such as tributyl phosphate and triphenyl phosphate; and acetate esters such as glycery triacetate and 2-ethylhexyl acetate. Examples of the flowing regulator include silicone-modified acrylate such as "TEGO(R) Rad 2100" (manufactured by Evonik Industries AG).

<Optical film>

The polymerizable liquid crystal composition according to the present embodiment can be used as a raw material of optical films such as a light reflection film, a retardation film, and a polarization film. Such optical films can be produced by forming an applied film of the polymerizable liquid crystal composition on a predetermined film used as a substrate, and curing the applied film. For example, when the light reflection film is produced by using the polymerizable liquid crystal composition, the polymerizable liquid crystal composition containing the aforementioned components is applied on a substrate (plastic film) such as a triacetylcellulose (TAC) film, an acryl film, a polycarbonate film, a polyvinyl chloride film, a polyolefin film, a polyethylene terephthalate (PET) film, etc. so that the thickness is as uniform as possible, and left to stand for a certain time while removing the solvent by heating to form the applied film on the substrate. The heating temperature is not particularly limited as long as it is temperature at which the polymerizable liquid crystal compound forms the cholesteric liquid crystal state to be oriented with the desired helical pitch on the substrate, and preferably 40 to 90°C. In this time, the orientation of the cholesteric liquid crystal can be more uniform by subjecting the surface of the substrate to an orientation treatment such as rubbing and stretching before applying the polymerizable liquid crystal composition, and a haze value as the optical film can be reduced. Then, while keeping this orientation state, the orientation of the cholesteric liquid crystal is fixed by irradiation of ultraviolet ray with a high-pressure mercury lamp etc. to produce the optical film (light reflection film) having a cured film of the polymerizable liquid crystal composition. Here, when the atropisomer exhibiting clockwise helical orientation is used, an optical film that selectively reflects right circularly polarized light is obtained. Meanwhile, when the atropisomer exhibiting counterclockwise helical orientation is used, an optical film that selectively reflects left circularly polarized light is obtained. This phenomenon of selectively reflecting the specific circularly polarized light is called

"selective reflection", and the optical film having the selective reflection function can be obtained.

The optical film according to the present embodiment exhibits the selective reflection function, and therefore, can reduce an incident light quantity and glare, and can reflect light within a specific wavelength band. The optical film is effectively applied for, for example: eyewear such as glasses, sunglasses, and goggles; and image display devices such as a liquid crystal display device and an organic EL display device.

Examples

Hereinafter, Examples of the present invention will be described, but the present invention is not limited to these examples unless departing from the spirit. Unless otherwise mentioned, "%" and "parts" hereinafter are each based on mass, and room temperature is within a range of 20°C±5°C.

<Example 1>

Into a reaction vessel containing 50 ml of dichloromethane (DCM), 1.43 g of (S)-1,1'-bi-2-naphthol (5 mmol: S-Binol), 61 mg of dimethylaminopyridine (DMAP), and 4.33 g of 4-({6-[(2-methylprop-2-enoyl)oxy]naphthalene-2-carbonyl} oxy)benzoic acid (11.5 mmol: Ma-NP-BA) were added, and stirred at room temperature. Into the obtained suspension liquid, 2.22 g of 1-(3-dimethylaminopropyl)-3-ethylcarbodiimide hydrochloride (11.5 mmol: EDC) was added separately in two times at each 1.11 g with an interval of 1 hour, and stirred for 20 hours. Thereafter, analysis by thin-layer chromatography (TLC) showed complete conversion of S-Binol into a diester. For workup, this solution was washed with a 10% sodium chloride solution to remove an EDC-urea byproduct, and dried with anhydrous magnesium sulfate. Then, the solution containing DCM was concentrated to about 30% (based on a maximum yield of Compound 2.1), and precipitated with 250 ml of methanol. The precipitate was filtered, then washed twice with methanol, and dried to obtain 4.49 g of a compound represented by the following general formula (2.1) (yield: 90%).

[Formula 15]

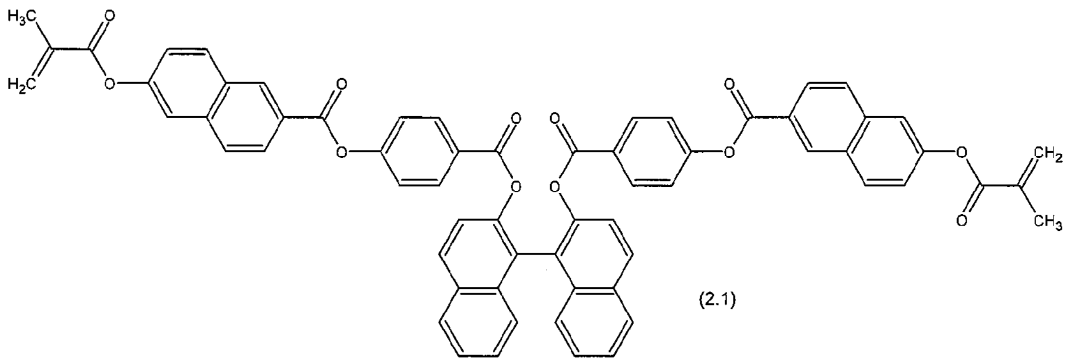

(2.1)

The compound represented by the general formula (2.1) (Compound 2.1) without further purification was added into a polymerizable liquid crystal compound (LC242 : RM257 = 1:1) containing 1.9% of a photopolymerization initiator (Omnirad TPO) and 0.1% of a flowing regulator (TEGO(R) Rad 2100) to regulate the content of Compound 2.1 relative to the polymerizable liquid crystal compound to 3.5%, and the obtained mixture of the polymerizable liquid crystal compound and Compound 2.1 was dissolved in m-xylene (solvent) to produce a polymerizable liquid crystal composition containing 3.5% of Compound 2.1 at 35%. This polymerizable liquid crystal composition was applied on a PET film (thickness: 100 μm) manufactured by TOYOBO Co., Ltd. and subjected in advance to a rubbing treatment with nylon cloth by spin-coating (number of rotation: 750 rpm). The obtained applied film was dried at 80°C for 5 minutes to orient the liquid crystal, and the applied film was irradiated with an LED (100 $mW/cm^2$) emitting light at a wavelength of 365 nm while purging nitrogen gas, and the applied film was cured at 80°C for 30 seconds to produce an optical film in which a cholesteric liquid crystal layer was formed on the PET film. This optical film had a center reflection wavelength of 494 nm, and Compound 2.1 had an HTP value of 92.5 $\mu m^{-1}$.

<Example 2>

(Step 1)

Into a reaction vessel containing 60 ml of DCM, 2.86 g of S-Binol (10 mmol), 0.12 g of DMAP, and 2.81 g of

4-(trans-4-pentylcyclohexyl)benzoic acid (10.25 mmol: 5PCA) were added, and stirred at room temperature. Into the obtained suspension liquid, 2.12 g of EDC (11 mmol) was added separately in two times at each 1.06 g with an interval of 1 hour, and stirred for 20 hours. Thereafter, analysis by thin-layer chromatography (TLC) showed formation of a monoester of S-Binol, a trace amount of S-Binol, and a trace amount of a diester of S-Binol ("byproduct").

(Step 2)

Into the solution obtained in Step 1 and containing the S-Binol monoester of 5PCA, 3.17 g of 4'-[(2-methylprop-2-enoyl)oxy][1,1'-biphenyl]-4-carboxylic acid (11.25 mmol: Ma-BPh-A) and 2.30 g of EDC (12 mmol) were added separately in two times at each 1.15 g with an interval of 1 hour, and stirred for 20 hours. Thereafter, analysis by thin-layer chromatography (TLC) showed complete conversion of S-Binol into a diester. For workup, this solution was washed with a 10% sodium chloride solution to remove an EDC-urea byproduct, and dried with anhydrous magnesium sulfate. Then, the solution containing DCM was concentrated to about 30% (based on a maximum yield of Compound 2.2), and precipitated with 300 ml of methanol. The precipitate was filtered, then washed twice with methanol, and dried to obtain 6.68 g of a compound represented by the following general formula (2.2) (yield: 83%).

[Formula 16]

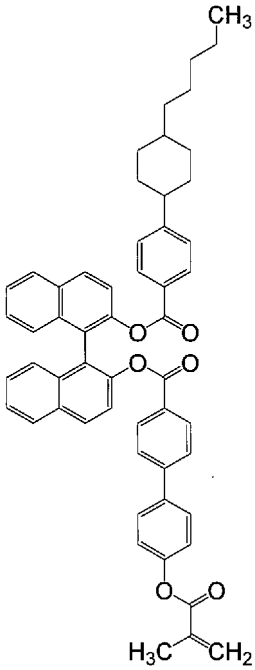

(2. 2)

The obtained compound represented by the general formula (2.2) (Compound 2.2) was not further purified, and an optical film was produced in the same manner as in Example 1 except that the content of Compound 2.2 relative to the polymerizable liquid crystal compound was regulated to 5.0%. This film had a center reflection wavelength of 416 nm, and Compound 2.2 had an HTP value of 76.9 $\mu m^{-1}$.

Compounds produced in the following Examples 3 to 9 were synthesized in two-stage step same as the production method described in Example 2.

<Example 3>

Used was 2.86 g of S-Binol (10 mmol) and reacted with 4-[(4-methoxybenzoyl)oxy]benzoic acid (MBBA) instead of 4-(trans-4-pentylcyclohexyl)benzoic acid in Step 1, and 4'-(2-methylprop-2-enoyl)oxy][1,1'-biphenyl]-4-carboxylic acid (Ma-BPh-A) was similarly used in Step 2. After workup, 7.8 g of a compound represented by the following general formula (2.3) was obtained (yield: 97%). The obtained compound represented by the general formula (2.3) (Compound 2.3) was not further purified, and an optical film was produced in the same manner as in Example 1 except that the content of Compound 2.3 relative to the polymerizable liquid crystal compound was regulated to 4.0%. This film had a center reflection wavelength of 493 nm, and Compound 2.3 had an HTP value of 81.1 $\mu m^{-1}$.

[Formula 17]

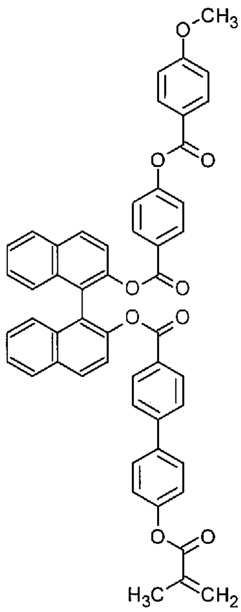

(2.3)

<Example 4>

Used was 2.86 g of S-Binol (10 mmol) and reacted with a benzoic acid corresponding to a compound represented by the (R-7) (5PCA-BA) instead of 4-(trans-4-pentylcyclohexyl)benzoic acid in Step 1, and 4'-(2-methylprop-2-enoyl)oxy][1,1'-biphenyl]-4-carboxylic acid (Ma-BPh-A) was similarly used in Step 2. After workup, 4.1 g of a compound represented by the following general formula (2.4) was obtained (yield: 97%). The obtained compound represented by the general formula (2.4) (Compound 2.4) was not further purified, and an optical film was produced in the same manner as in Example 1 except that the content of Compound 2.4 relative to the polymerizable liquid crystal compound was regulated to 3.5%. This film had a center reflection wavelength of 545 nm, and Compound 2.4 had an HTP value of 83.9 $\mu m^{-1}$.

[Formula 18]

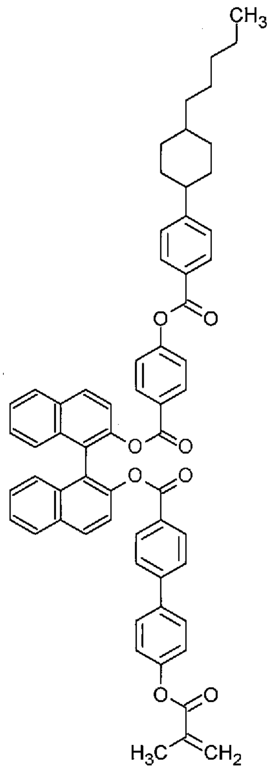

(2.4)

<Example 5>

Used was 2.86 g of S-Binol (10 mmol) and reacted with a benzoic acid corresponding to a compound represented by the (R-7) (5PCA-BA) instead of 4-(trans-4-pentylcyclohexyl)benzoic acid in Step 1, and a benzoic acid corresponding to a compound represented by the (R-3) (Ma-BPh-BA) was used instead of 4'-(2-methylprop-2-enoyl)oxy][1,1'-biphenyl]-4-carboxylic acid in Step 2. After workup, 10.1 g of a compound represented by the following general formula (2.5) was obtained (yield: 97%). The obtained compound represented by the general formula (2.5) (Compound 2.5) was not further purified, and an optical film was produced in the same manner as in Example 1 except that the content of Compound 2.5 relative to the polymerizable liquid crystal compound was regulated to 3.0%. This film had a center reflection wavelength of 548 nm, and Compound 2.5 had an HTP value of 97.3 $\mu m^{-1}$.

[Formula 19]

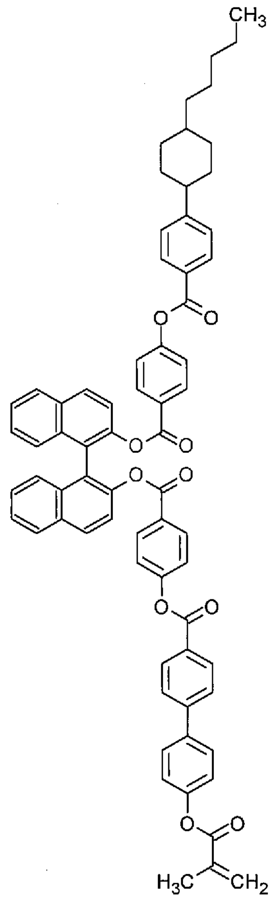

**(2.5)**

<Example 6>

Used was 2.86 g of S-Binol (10 mmol) and reacted with 4-(trans-4-pentylcyclohexyl)benzoic acid (5PCA) in Step 1, and a benzoic acid corresponding to a compound represented by the (R-3) (Ma-BPh-BA) was used instead of 4'-(2-methylprop-2-enoyl)oxy][1,1'-biphenyl]-4-carboxylic acid in Step 2. After workup, 7.3 g of a compound represented by the following general formula (2.6) was obtained (yield: 78%). The obtained compound represented by the general formula (2.6) (Compound 2.6) was not further purified, and an optical film was produced in the same manner as in Example 1 except that the content of Compound 2.6 relative to the polymerizable liquid crystal compound was regulated to 3.5%. This film had a center reflection wavelength of 546 nm, and Compound 2.6 had an HTP value of 83.7 $\mu m^{-1}$.

[Formula 20]

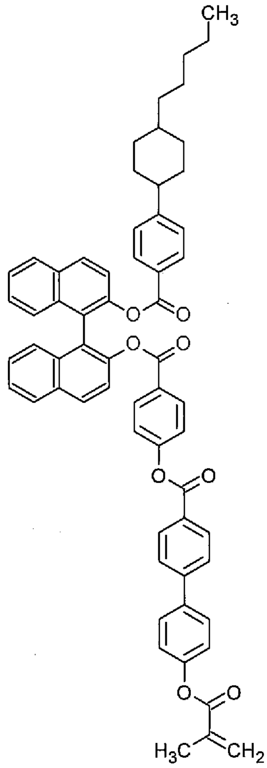

**(2.6)**

<Example 7>

Used were 2.86 g of S-Binol (10 mmol) and 61 mg of DMAP, reacted with 4-(trans-4-propylcyclohexyl)benzoic acid (3PCA) instead of 4-(trans-4-pentylcyclohexyl)benzoic acid in Step 1, and 4-({6-[(2-methylprop-2-enoyl)oxy] naphthalene-2-carbonyl}oxy)benzoic acid (Ma-NP-BA) was used instead of 4'-(2-methylprop-2-enoyl)oxy][1,1'-biphenyl]-4-carboxylic acid in Step 2. After workup, 7.4 g of a compound represented by the following general formula (2.7) was obtained (yield: 85%). The obtained compound represented by the general formula (2.7) (Compound 2.7) was not further purified, and an optical film was produced in the same manner as in Example 1 except that the content of Compound 2.7 relative to the polymerizable liquid crystal compound was regulated to 3.5%. This film had a center reflection wavelength of 572 nm, and Compound 2.7 had an HTP value of 79.9 $\mu m^{-1}$.

[Formula 21]

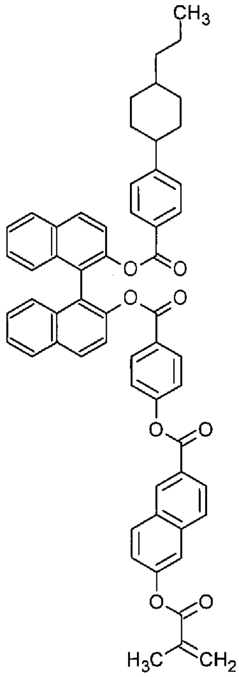

(2.7)

<Example 8>

Used was 2.86 g of (R)- 1,1'-bi-2-naphthol (10 mmol: R-Binol) and reacted with a benzoic acid corresponding to the compound represented by the (R-7) (5PCA-BA) instead of 4-(trans-4-pentylcyclohexyl)benzoic acid in Step 1, and 4-({6-[(2-methylprop-2-enoyl)oxy]naphthalene-2-carbonyl}oxy)benzoic acid (Ma-NP-BA) was used instead of 4'-(2-methylprop-2-enoyl)oxy][1,1'-biphenyl]-4-carboxylic acid in Step 2. After workup, 10.0 g of a compound represented by the following general formula (2.8) was obtained (yield: 98%). The obtained compound represented by the general formula (2.8) (Compound 2.8) was not further purified, and an optical film was produced in the same manner as in Example 1 except that the content of Compound 2.8 relative to the polymerizable liquid crystal compound was regulated to 3.5%. This film had a center reflection wavelength of 495 nm, and Compound 2.8 had an HTP value of 92.4 $\mu m^{-1}$.

[Formula 22]

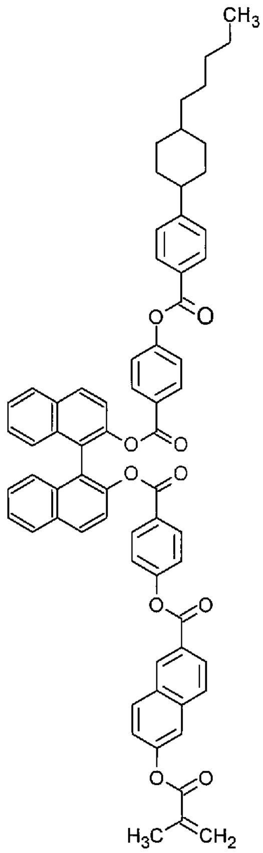

**(2.8)**

<Example 9>

Used were 21.45 g of S-Binol (75 mmol) and 0.46 g of DMAP, reacted with 4-(trans-4-propylcyclohexyl)benzoic acid (3PCA) instead of 4-(trans-4-pentylcyclohexyl)benzoic acid in Step 1, and 4'-[(2-methylprop-2-enoyl)oxy][1,1'-biphenyl]-4-carboxylic acid was used in Step 2. After workup, 53.1 g of a compound represented by the following general formula (2.9) was obtained (yield: 91%). The obtained compound represented by the general formula (2.9) (Compound 2.9) was not further purified, and an optical film was produced in the same manner as in Example 1 except that the content of Compound 2.9 relative to the polymerizable liquid crystal compound was regulated to 3.5%. This film had a center reflection wavelength of 570 nm, and Compound 2.9 had an HTP value of 80.2 $\mu m^{-1}$.

[Formula 23]

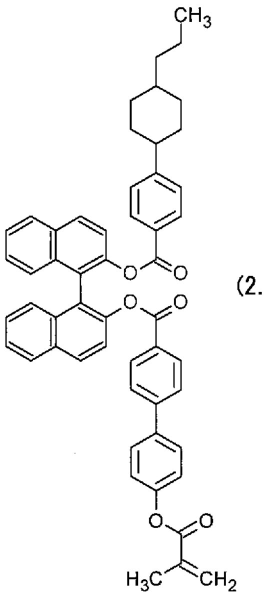

(2. 9)

<Comparative Example 1>

Instead of the atropisomer of the present invention, a commercial product "Lumogen(R) S750" (manufactured by BASF SE) was used as a comparative compound. An optical film was produced in the same manner as in Example 1 except that the content of the comparative compound relative to the polymerizable liquid crystal compound was regulated to 5.0%. This film had a center reflection wavelength of 509 nm, and the comparative compound had an HTP value of 62.9 $\mu m^{-1}$.

As for the atropisomers produced in Examples 1 to 9 and the comparative compound used in Comparative Example 1, and each of the optical films, an HTP value and a center reflection wavelength were measured as follows while changing the content (hereinafter, which may be referred to as "concentration") of the atropisomer relative to the polymerizable liquid crystal compound. Table 1 shows the results.

<HTP value and center reflection wavelength>

Each of Compounds 2.1 to 2.9 and the comparative compound (dopant agents) was added into the polymerizable liquid crystal compound (LC242 : RM257 = 1:1) to regulate the concentration of the dopant agent relative to the polymerizable liquid crystal compound, and an absolute value of the HTP value was calculated based on the following expression (A). In the expression (A), P represents a helical pitch length ($\mu$m), and C represents the concentration (%) of the dopant agent relative to the polymerizable liquid crystal compound. P was calculated based on the following expression (B) by measuring a center reflection wavelength ($\lambda_r$) in a reflection band of the optical film. Here, an average refractive index (n) of the polymerizable liquid crystal compound was presumed to be 1.6 for the calculation. The center reflection wavelength ($\lambda_r$) was specified by measuring a reflection spectrum of the optical film using a spectrometer, detecting wavelengths ($\lambda_{min}$ and $\lambda_{max}$) corresponding to a half of the maximum height of the reflection spectrum, and calculating $(\lambda_{min}+\lambda_{max})/2$.
[Expression 1]

$$HTP = \frac{1}{P \cdot C} \quad \cdots \text{(A)}$$

[Expression 2]

$$P = \lambda_r / n \quad \cdots \text{(B)}$$

[Table 1]

| | Atropisomer / comparative compound | | | | | | Center reflection wavelength 180°C UV irradiation [nm] | | | | HTP / n=1.6 [μm - 1] | | | |
|---|---|---|---|---|---|---|---|---|---|---|---|---|---|---|
| | R/S | $R_1$ | $R_2$ | Molecular weight | Symmetrical structure | Mono / di-acryl | 5.0% | 4.0% | 3.5% | 3.0% | 5.0% | 4.0% | 3.5% | 3.0% |
| Example 1 | S | R-1 | R-1 | 1002 | Symmetrical | Diacryl | | 435 | 494 | 577 | | 92.0 | 92.5 | 92.4 |
| Example 2 | S | R-6b | R-4 | 806 | Unsym metrical | Monoacryl | 416 | 505 | | 655 | 76.9 | 79.2 | | 81.4 |
| Example 3 | S | R-9 | R-4 | 804 | Unsym metrical | Monoacryl | 405 | 493 | | 651 | 79.0 | 81.1 | | 81.9 |
| Example 4 | S | R-7 | R-4 | 926 | Unsym metrical | Monoacryl | | | 545 | | | | 83.9 | |
| Example 5 | S | R-7 | R-3 | 1046 | Unsym metrical | Monoacryl | 344 | 418 | | 548 | 93.0 | 95.7 | | 97.3 |
| Example 6 | S | R-6b | R-3 | 926 | Unsym metrical | Monoacryl | | | 546 | | | | 83.7 | |
| Example 7 | S | R-6a | R-1 | 872 | Unsym metrical | Monoacryl | | | 572 | | | | 79.9 | |
| Example 8 | R | R-7 | R-1 | 1020 | Unsym metrical | Monoacryl | | | 495 | | | | 92.4 | |
| Example 9 | S | R-6a | R-4 | 778 | Unsym metrical | Monoacryl | | | 570 | | | | 80.2 | |
| Comparative Example 1 | S | - | - | | Symmetrical | Diacryl | 509 | 631 | | | 62.9 | 63.4 | | |

* R-6a and R-6b corresponded to the structure (R-6) in which A = n-propyl group and A = pentyl group, respectively.

As shown in Table 1, all the atropisomers produced in Examples 1 to 9 exhibited the HTP value of more than 75 even with changing the addition amount within a range of 3 to 5%, and exhibited the high HTP values even with the small amount. In particular, the atropisomers produced in Examples 1 and 5 exhibited extremely high HTP values of more than 90 even with the small addition amount of 3%. In addition, since the optical films produced in Examples 1 to 6 can change the center reflection wavelength depending on the addition amount even with the small addition amount, the optical film with broadened reflection band in the visible light region has been obtained without affecting the properties of the liquid crystal.

Meanwhile, the comparative compound used in Comparative Example 1 exhibited a lower HTP value than that the atropisomers produced in Examples 1 to 9 had. In addition, the center reflection wavelength of the optical film produced in Comparative Example 1 was respectively 509 nm and 630 nm with the addition amount of 5.0% and 4.0%, and the reflection band in the visible light region with a lower concentration was limited.

Industrial Applicability

The present invention can provide the atropisomer having the high HTP value even with a small amount, and the polymerizable liquid crystal composition, the optical film, the image display device, and the eyewear using the same.

## Claims

1. An atropisomer of a compound represented by the general formula (1) and/or the general formula (2):

[Formula 1]

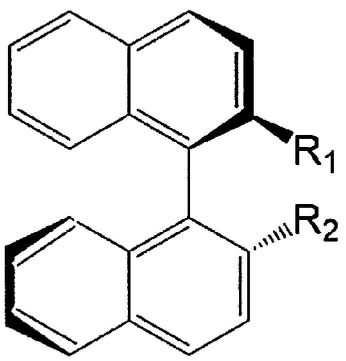
(1)

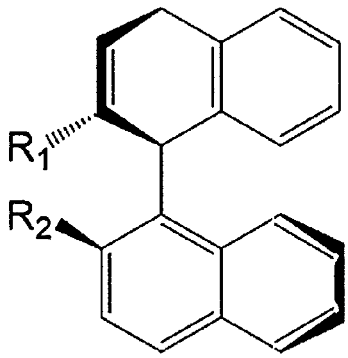
(2)

wherein $R_1$ and $R_2$ each independently represent any one structure of (R-1) to (R-20), and at least one of $R_1$ and $R_2$ represents any one of (R-1) to (R-5), provided that $R_1$ and $R_2$ are not same except for a case where the structure is (R-1),

[Formula 2]

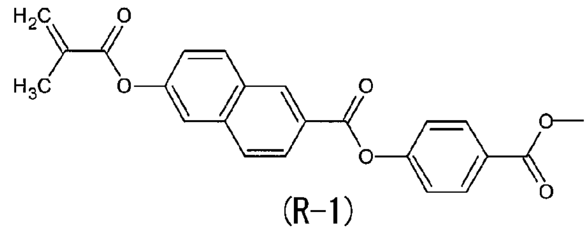

(R-1)

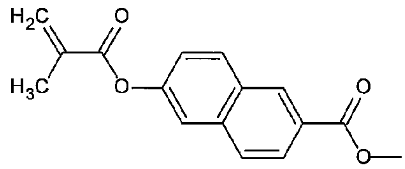

(R-2)

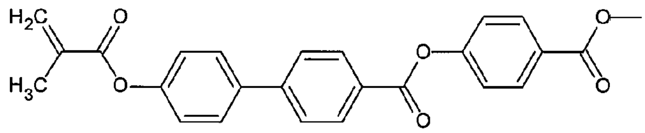

(R-3)

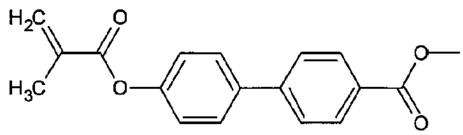

(R-4)

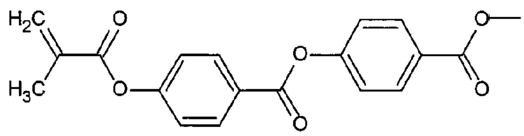

(R-5)

[Formula 3]

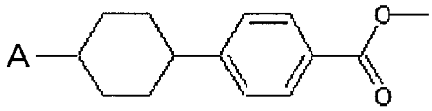

(R-6)

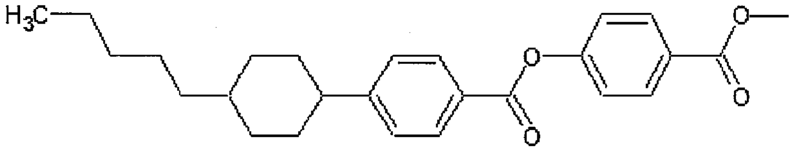

(R-7)

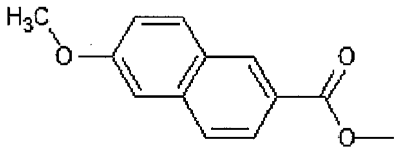

(R-8)

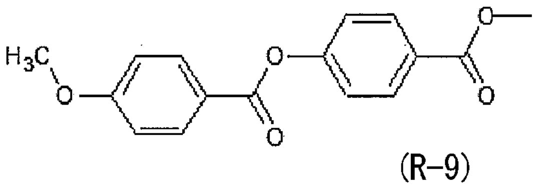

(R-9)

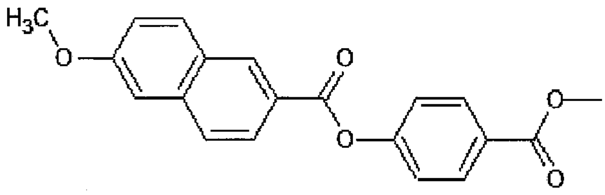

(R-10)

[Formula 4]

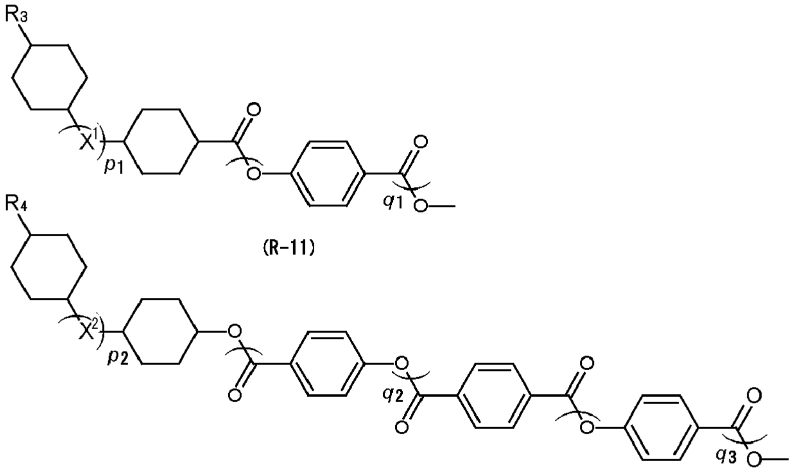

(R-11)

(R-12)

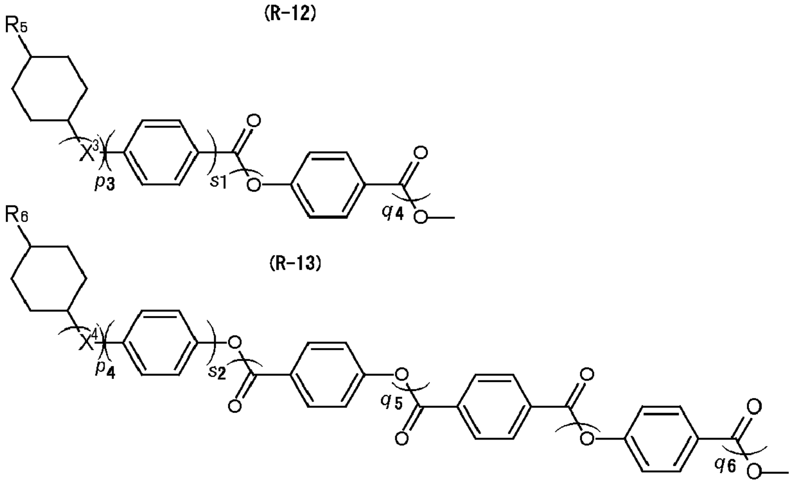

(R-13)

(R-14)

[Formula 5]

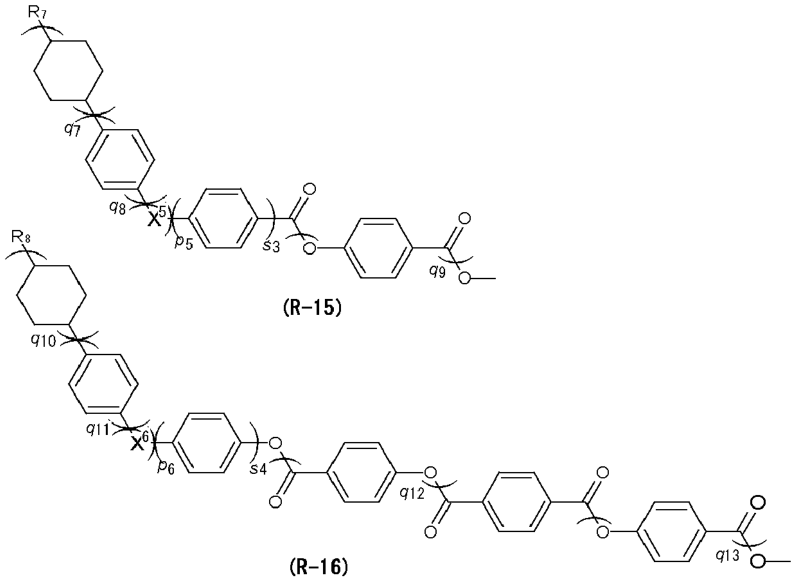

(R-15)

(R-16)

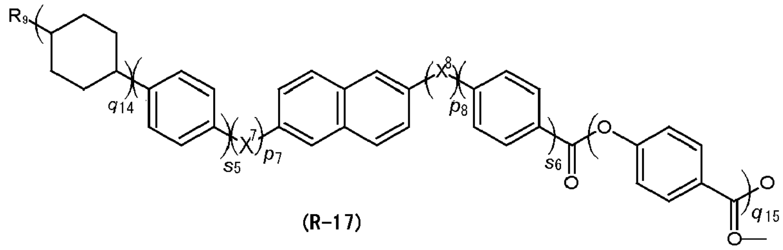

(R-17)

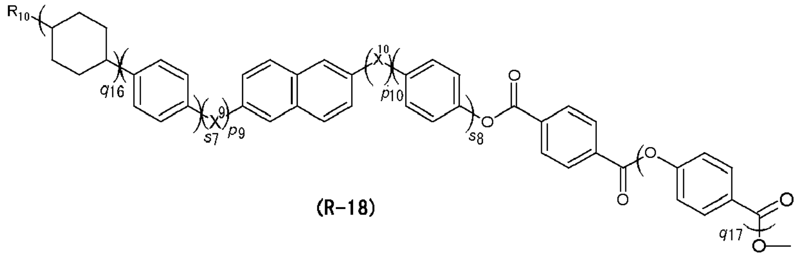

(R-18)

[Formula 6]

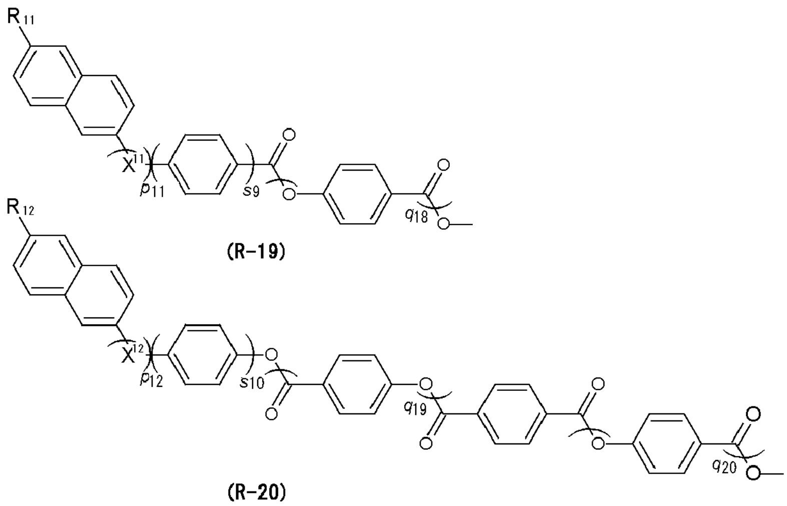

(R-19)

(R-20)

wherein A represents a $C_{1-12}$ alkyl group,

$R_3$ to $R_{12}$ each independently represent $-CnH_{2n+1}$, $-OC_nH_{2n+1}$, CN, F, Cl, Br, $CF_3$, $Y-((CH_2)_{k1}-O)_m-$, $Y-(CH_2)_{k2}-O-C(O)-$, or $Y-(CH_2)_{k3}-COO-$,

Y represents $H_2C=C(Z_1)COO-$, $(H_2COCH)-CH_2-$, $H_2C=CH-CH_2-$, or $H_2C=CH-O-$,
$Z_1$ represents H or $CH_3$, k1 to k3 each independently represent an integer of 0 to 12,
m represents 0 or 1,
n represents an integer of 0 to 12,
$X^1$ to $X^{12}$ each independently represent -CH=CH-, -C≡C-, -HC=CH-COO-, -(O)CO-CH=CH-, $-CH_2-$, -O-, -C(O)-, $-CH_2CH_2-$, $-CH_2O-$, $-OCH_2-$, -COO-, - OC(O)-, $-C(CH_3)_2-$, -N=N-, -CH=N-, -N=CH-, -NHCO-, or -OCNH-,
p1 to p12 each independently represent 0 or 1,
q1 to q20 each independently represent 0, 1, or 2, and
s1 to s10 each independently represent 0, 1, 2, or 3.

**2.** The atropisomer according to claim 1, wherein $R_1$ and $R_2$ each independently represent any one structure of (R-1) to (R-10), and at least one of $R_1$ and $R_2$ represents any one of (R-1) to (R-4).

**3.** A polymerizable liquid crystal composition, comprising:

the atropisomer according to claim 1 or 2; and
a polymerizable liquid crystal compound.

**4.** An optical film, comprising a cured film of the polymerizable liquid crystal composition according to claim 3.

**5.** The optical film according to claim 4, wherein the optical film has a selective reflection function.

**6.** An image display device, comprising the optical film according to claim 4.

**7.** Eyewear, comprising the optical film according to claim 4.

| INTERNATIONAL SEARCH REPORT | International application No. PCT/JP2023/020248 |
|---|---|

**A. CLASSIFICATION OF SUBJECT MATTER**

***C08F 20/30***(2006.01)i; ***C07C 69/54***(2006.01)i; ***C09K 19/58***(2006.01)i; ***G02B 5/30***(2006.01)i; ***G02C 7/10***(2006.01)i; ***G02C 7/12***(2006.01)i
FI: C08F20/30; C07C69/54 Z; C09K19/58; G02B5/30; G02C7/12; G02C7/10

According to International Patent Classification (IPC) or to both national classification and IPC

**B. FIELDS SEARCHED**

Minimum documentation searched (classification system followed by classification symbols)

C08F20/30; C07C69/54; C09K19/58; G02B5/30; G02C7/10; G02C7/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2018/194157 A1 (FUJIFILM CORP.) 25 October 2018 (2018-10-25) entire text | 1-7 |
| A | JP 2015-110728 A (JNC CORP.) 18 June 2015 (2015-06-18) entire text | 1-7 |
| A | JP 2015-135474 A (JNC CORP.) 27 July 2015 (2015-07-27) entire text | 1-7 |
| A | GUO, Renwei et al. Chiral polymer networks with a broad reflection band achieved with varying temperature. Polymer. 2010, 51, 5990-5996, fig. 1 fig. 1 | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2023** | **25 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/020248**

| Patent document cited in search report | Publication date (day/month/year) | Patent family member(s) | Publication date (day/month/year) |
|---|---|---|---|
| WO 2018/194157 A1 | 25 October 2018 | US 2020/0071615 A1<br>entire text | |
| JP 2015-110728 A | 18 June 2015 | US 2015/0108401 A1<br>entire text<br>KR 10-2015-0068892 A | |
| JP 2015-135474 A | 27 July 2015 | US 2016/0011349 A1<br>entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013087109 A
- JP 2003315556 A
- JP 2004029824 A
- JP 5463666 B